# EUROPEAN PATENT SPECIFICATION

(11) **EP 2 612 159 B1**
(45) Date of publication and mention of the grant of the patent: **11.03.2020**
(21) Application number: 11760570.9
(22) Date of filing: 24.08.2011
(51) Int. Cl.: G01R 33/28, G01R 33/30, A61M 16/01, G01R 33/56

(54) **MAGNETIC RESONANCE IMAGING SYSTEM, COMPUTER SYSTEM, AND COMPUTER PROGRAM PRODUCT FOR SENDING CONTROL MESSAGES TO AN ANESTHESIA SYSTEM**
MAGNETRESONANZ-BILDGEBUNGSSYSTEM, COMPUTERSYSTEM UND COMPUTERPROGRAMMPRODUKT ZUM SENDEN VON STEUERUNGSNACHRICHTEN AN EIN ANÄSTHESIESYSTEM
SYSTÈME D'IMAGERIE PAR RÉSONANCE MAGNÉTIQUE, SYSTÈME INFORMATIQUE, ET PRODUIT DE PROGRAMME INFORMATIQUE POUR ENVOYER DES MESSAGES DE COMMANDE À UN SYSTÈME D'ANESTHÉSIE

(30) Priority: 30.08.2010 EP 10174463
(43) Date of publication of application: 10.07.2013
(73) Proprietor: Koninklijke Philips N.V., 5656 AE Eindhoven (NL); Philips Intellectual Property & Standards GmbH, 20099 Hamburg (DE)
(72) Inventor: REMMELE, Stefanie, NL-5656 AE Eindhoven (NL); STEHNING, Christian, NL-5656 AE Eindhoven (NL)
(74) Representative: van Velzen, Maaike Mathilde
(86) International application number: PCT/IB2011/053717
(87) International publication number: WO 2012/028997

(56) References cited:
- US-A1- 2004 230 113
- HEDLUND L W ET AL: "MR-COMPATIBLE VENTILATOR FOR SMALL ANIMALS: COMPUTER-CONTROLLED VENTILATION FOR PROTIN AND NOBLE GAS IMAGING", MAGNETIC RESONANCE IMAGING, ELSEVIER SCIENCE, TARRYTOWN, NY, US, vol. 18, no. 6, 1 January 2000 (2000-01-01), pages 753-759, XP001029472, ISSN: 0730-725X, DOI: DOI:10.1016/S0730-725X(00)00154-5 cited in the application
- OGAWA S ET AL: "Brain magnetic resonance imaging with contrast dependent on blood oxygenation", PROCEEDINGS OF THE NATIONAL ACADEMY OF SCIENCES OF THE UNITED STATES (PNAS), NATIONAL ACADEMY OF SCIENCE, US, vol. 87, no. 24, 1 December 1990 (1990-12-01), pages 9868-9872, XP002554038, ISSN: 0027-8424, DOI: DOI:10.1073/PNAS.87.24.9868
- ZHAO P ET AL: "Vasoreactivity and peri-infarct hyperintensities in stroke", NEUROLOGY, vol. 72, 2009, pages 643-649, XP002610859,
- MARK CI ET AL: "Precise control of end-tidal carbon dioxide and oxygen improves BOLD and ASL cerebrovascular reactivity measures", MAGNETIC RESONANCE IN MEDICINE, ACADEMIC PRESS, DULUTH, MN, US, vol. 64, 20 July 2010 (2010-07-20), pages 749-756, XP002610860,

## Description

### TECHNICAL FIELD

The invention relates to magnetic resonance imaging, in particular to the simultaneous control of a magnetic resonance imaging system and an anesthesia system

### BACKGROUND OF THE INVENTION

Measuring the Magnetic Resonance (MR) response to Respiratory Challenges (RC) that induce elevated levels of O2 (hyperoxia) and CO2 (hypercapnia) gives insight into a wide range of physiologic parameters like blood and tissue oxygenation, vessel maturation and function, tumor hypoxia and cerebrovascular reactivity. These parameters play a major role in oncologic research e.g. in tumor therapy selection, planning (dosage) and monitoring. In those experiments, the patient inhales O2 and/or CO2-enriched air, which results in a modulation of blood flow, volume and oxygenation depending on the maturity and oxygen function of vessels and/or the amount of dissolved oxygen in blood plasma and tissue. In response, MR relaxation rates and thus the MR contrast changes, according to the impact of the challenge on the magnetic properties of tissue and blood and the relaxation parameters, respectively (oxygenated blood is diamagnetic increasing T2*, dissolved oxygen in blood and tissue is paramagnetic, decreasing T1, accelerated inflow during enhanced CO2 levels decreases T1 artificially, etc.).

In journal article Mandell et. al., "Selective Reduction of Blood Flow to White Matter During Hypercapnia" Stroke, DOI:10.1161/STROKEAHA.107.501692 an automated gas sequence was used to alternate between high and low end-tidal pressure of carbon dioxide states during a BOLD magnetic resonance acquisition.

In the paper by L.W. Hedlund et al., 'MR compatible ventilator for small animals: computer controlled ventilation for proton and noble gas imaging' in Magnetic Resonance Imaging 18(2000)753-759, it is mentioned that ventilation and imaging can be synchronised by having either the ventilator actively trigger imaging or by having the imaging trigger the ventilator.

US 2004/230113 A1 discloses a method for using of polarized inert noble gases such as helium and xenon for contrast enhanced magnetic resonance imaging using a ventilator system with a trigger input/output port that can generate gated trigger signals to an NMR/ MRI system during or prior to signal acquisition.

### SUMMARY OF THE INVENTION

The invention provides for a magnetic resonance imaging system, a computer system, and a computer program product in the independent claims. Embodiments are given in the dependent claims.

A difficulty with current Respiratory Challenge (RC) procedures is that the conventional regime comprises one or more MR scans, executed while the patient is inhaling changing compositions of O2, CO2 or inhalation of other inhalation gases, also known as a RC, units that are controlled completely separately and independently from the MR unit. In other words, the radiologist and anesthetist agree on a particular RC protocol (e.g. 1min normal air, 4 min oxygen, 2 min normal air). Then, the MR scan(s) and the delivery and mixture of inhalation gases are synchronized manually and the exact timing of the truly applied breathing regime with respect to the MR scan has to be recorded manually for later analysis of the MR data.

Problems arise, when the MR scan had not been perfectly synchronized to the RC protocol or vice versa, e.g. due to:
- misunderstandings of technicians and radiologists outside the MR room with the anesthetist inside the MR room,
- timing uncertainties,
- leakage of the inhalation mask, via which the patient inhales the applied mixtures, or
- post-processing fails, when the (manual) RC protocol recording gets lost or is incorrect.

For example, if the parameterized description of the MR response (i.e. the MR signal over time or relaxation constants over time) to the delivery of increased O2, CO2 levels reveals markers of physiologic tumor parameters (oxygenation, vasoreactivity). For this, a signal model is fitted to the recorded series of images. This model may be either obtained experimentally or is based on a tissue compartment model. Numerical fit routines require the appropriate initialization of the model parameters. These serve as start values of an iterative approximation of the true parameters. Depending on the algorithm, the fit result will be more or less sensitive to a wrong initialization of fit parameters, e.g. if a wrong RC protocol was communicated to the MR processing. For example, an anesthetist applies a RC protocol to the patient, which comprises 1/4/2min of breathing air/Carbogen/air (Carbogen is a composition of O2 and CO2). Later on, during data analysis, the radiologist or MR technician enters the wrong RC protocol (2/4/1min) into the postprocessing module (typing error, misinformation, mixing up of the two "air" periods, etc.).

The invention involves a communication link module between the MR environment and the RC environment replacing the "manual" communication between the MR and RC operators to:
- perfectly synchronize the MR scan(s) and the respiratory challenge
- to ensure accurate analysis of the MR data (e.g. if a signal model is fitted to a dynamic series of images recorded during inhalation changes, this mostly requires knowledge about when the challenge started/changed/ended)..

The interest of MR imaging during O2 and CO2 respiratory challenges, has significantly grown and is still growing especially in oncology. The conventional "manual" synchronization of respiratory and MR scan protocols is particularly difficult in the MR environment, the communication between the MR operator and the RC operator (anesthetist) is prone to misunderstandings, which translates into systematic errors in the analysis and interpretation of the MR data. The use of a synchronized and automated communication link between the MR and RC environment overcomes these issues and ensures an optimized and robust use of the experiment and will thus considerably improve this powerful diagnostic tool. Product implementation of oxygen or CO2 enhanced MRI necessitates at least the installation of some means for synchronization of the two environments to guarantee robustness (user independence). This is covered by the present invention.

A 'computer-readable storage medium' as used herein is any storage medium which may store instructions which are executable by a processor of a computing device. The computer-readable storage medium may be a computer-readable non-transitory storage medium. The computer-readable storage medium may also be a tangible computer readable medium. In some embodiments, a computer-readable storage medium may also be able to store data which is able to be accessed by the processor of the computing device. An example of a computer-readable storage medium include, but are not limited to: a floppy disk, a magnetic hard disk drive, a solid state hard disk, flash memory, a USB thumb drive, Random Access Memory (RAM) memory, Read Only Memory (ROM) memory, an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network.

Computer memory is an example of a computer-readable storage medium. Computer memory is any memory which is directly accessible to a processor. Examples of computer memory include, but are not limited to: RAM memory, registers, and register files.

Computer storage is an example of a computer-readable storage medium. Computer storage is any non-volatile computer-readable storage medium. Examples of computer storage include, but are not limited to: a hard disk drive, a USB thumb drive, a floppy drive, a smart card, a DVD, a CD-ROM, and a solid state hard drive. In some embodiments computer storage may also be computer memory or vice versa.

A 'processor' as used herein encompasses an electronic component which is able to execute a program or machine executable instruction. References to the computing device comprising "a processor" should be interpreted as possibly containing more than one processor. The term computing device should also be interpreted to possibly refer to a collection or network of computing devices each comprising a processor. Many programs have their instructions performed by multiple processors that may be within the same computing device or which may even distributed across multiple computing device.

Magnetic resonance data is defined herein as being the recorded measurements of radio frequency signals emitted by atomic spins by the antenna of a Magnetic resonance apparatus during a magnetic resonance imaging scan. A Magnetic Resonance Imaging (MRI) image is defined herein as being the reconstructed two or three dimensional visualization of anatomic data contained within the magnetic resonance imaging data. This visualization can be performed using a computer.

In one aspect the invention provides for a magnetic resonance imaging system as defined in claim 1. The MRI system comprises a magnet adapted for generating a magnetic field for orienting the magnetic spins of nuclei of a subject located within an imaging volume. The magnetic resonance imaging system further comprises a radio frequency system for acquiring magnetic resonance data. The radio frequency system comprises a radio frequency transceiver adapted to connect to a radio frequency coil. As used herein a radio frequency transceiver may also refer to individual radio frequency transmitters and receivers. The radio frequency coil may also be separate transmit and receive coils. The magnetic resonance imaging system further comprises a magnetic field gradient coil for spatial encoding of magnetic spins and nuclei within the imaging volume. The magnetic resonance imaging system further comprises a magnetic field gradient coil power supply for supplying current to the magnetic field gradient coil. The magnetic resonance imaging system further comprises an anesthesia system interface adapted for sending control messages to an anesthesia system.

An 'anesthesia system' as used herein encompasses a system or apparatus adapted for controlling the flow of and for mixing gases which are provided for the subject. An anesthesia system is used for controlling the gas delivery of inhalation gases to a subject. For instance the concentration of oxygen and/or carbon dioxide may be controlled. The wording 'anesthesia system' is used because the anesthesia system is ideally supervised by an anesthesiologist.

The magnetic resonance imaging system further comprises a computer system comprising a processor and a memory. Memory contains instructions for execution by the processor. Execution of the instructions causes the processor to perform the step of controlling the operation of the magnetic resonance imaging system to acquire magnetic resonance data. Essentially the computer system performs as a control system for the magnetic resonance imaging system. Execution of the instructions further requires a processor to perform the step of sending control messages to the anesthesia system via the anesthesia system interface. The computer system essentially also functions as a control system for the anesthesia system. This is particularly advantageous because the computer system functions as a control system for both the magnetic resonance imaging system and for the anesthesia system. This allows more precise control of both apparatuses and allows their functionality to be more precisely synchronized. This enables the anesthesia system to be used for respiratory challenge studies using the magnetic resonance imaging system. By having the computer system control both apparatuses the magnetic resonance data which can be used to construct magnetic resonance images or other reconstructions is maybe more closely correlated with the respiratory challenge which is performed by the anesthesia system.

The computer memory contains a pulse sequence for planning the acquisition of magnetic resonance data. As used herein 'a pulse sequence' is a set of instructions or a timing plan for controlling the operation of the magnetic resonance imaging system. The instructions control the operation of the magnetic resonance imaging system in accordance with the pulse sequence. The memory comprises a gas sequence for planning the control of the composition of the gas delivered to the subject for respiration by the anesthesia system during the acquisition of magnetic resonance data. The instructions send control messages to the anesthesia system in accordance with the gas sequence.

The anesthesia system interface is further adapted for receiving gas sensor data from the anesthesia system. 'Gas sensor data' as used herein encompasses measurements from sensors within the anesthesia system for detecting properties of gas either inhaled or exhaled by the subject. The gas sensor data comprises time dependent gas concentrations in the gas inhaled and/or exhaled by the subject. The gas sensor data is time correlated with the magnetic resonance data in accordance with the pulse sequence. This is particularly advantageous because instead of purely relying on studying the gas flow to the subject the actual gas which is inhaled or exhaled can be measured using gas sensors which provide the gas sensor data. This allows any respiratory challenge procedure to be very precisely correlated with the pulse sequence. Correlating this with the pulse sequence allows the magnetic resonance images generated from the magnetic resonance data to be very accurately correlated with the portion of the respiratory challenge procedure.

In an embodiment the gas concentrations measured in the gas comprise oxygen concentrations.

In another embodiment the gas concentrations measured in the gas comprise carbon dioxide concentrations.

In another embodiment the gas concentrations measured in the gas comprise nitrogen concentrations.

According to the invention, the instructions further cause the processor to perform the step of analyzing the magnetic resonance data in accordance with a respiratory challenge algorithm. A respiratory challenge algorithm as used herein encompasses an algorithm which performs a statistical analysis of the magnetic resonance data in order to infer the effects of a respiratory challenge protocol. A respiratory challenge protocol as used herein encompasses altering the property of gases inhaled by a subject during a magnetic resonance imaging examination. The initialization of the respiratory challenge protocol algorithm is performed in accordance with the gas sensor data. Essentially the statistical analysis of the magnetic resonance data is correlated to the timing of data from the gas sensor data. For instance if carbon dioxide levels are elevated in the gas inhaled by the subject the respiratory challenge algorithm can be correlated to start when the elevated carbon dioxide levels are measured in the mask of the subject.

Execution of the instructions further cause a processor to perform the step of acquiring magnetic resonance data which may be reconstructed into tissue oxygenation level dependent contrast images. This may be accomplished for instance by measuring the so-called T2* or T1 or by instructing T2* or T1- weighted images. Execution of the instructions further causes the processor to perform the step of reconstructing the magnetic resonance data into tissue oxygenation level dependent contrast images. Execution of the instructions further causes the processor to perform the step of determining a set of tissue oxygenation level measures. For instance the tissue oxygenation level measures may be determined by the value of T2* or T1 or from T2*- or T1- weighted images. The set of tissue oxygenation level measures is constructed by determining a tissue oxygenation measure for an oxygenation test volume in each of the tissue oxygenation level dependent contrast images. The respiratory challenge algorithm analyses the magnetic resonance data by at least performing a statistical analysis of a subset of a set of tissue oxygenation level measures. The subset is determined in accordance with the gas sensor data.

In an embodiment each tissue oxygenation level measure is calculated during the acquisition of magnetic resonance data. According to the invention, the instructions further cause the processor to perform the step of modifying the gas sequence and, optionally, the pulse sequence in accordance with the set of tissue oxygen level measures. The gas sequence could be modified by measuring the effect of the tissue oxygen level measures. For instance, if a sufficient change in the tissue oxygen level is detected then the experiment could be terminated and the gas sequence could be modified such that the subject is breathing normal air. Depending on the contrast of the set of tissue oxygen level measures detected in magnetic resonance images the pulse sequence can be modified. For instance the repetition time and the number of echoes used for the T2* measurement may be modified.

In another embodiment the execution of the instructions further cause the processor to perform the step of acquiring magnetic resonance data which may be reconstructed into vasoreactivity contrast images. This may be accomplished for instance by acquiring magnetic resonance data which may be reconstructed into T1 weighted or T2*-weighted images or by measuring T2* or T1. The instructions further cause the processor to perform the step of reconstructing the magnetic resonance data into vasoreactivity contrast images. The vasoreactivity contrast images may for instance be T1 weighted contrast images. Execution of the instructions further requires a processor to perform the step of determining a set of vasoreactivity measures. The set of vasoreactivity measures is constructed by determining a vasoreactivity level for a vasoreactivity test volume in each of the vasoreactivity contrast images.

The RC protocols used for acquiring the magnetic resonance data which may be reconstructed into vasoreactivity contrast images or tissue oxygenation level dependent contrast images differ in by the use of different gasses. The analysis of the magnetic resonance data is either the same or similar. Information about both oxygenation and vasoreactivity can be drawn from T1, T2* or T2 weighted images or T1, T2* or T2 values.

The respiratory challenge algorithm analyses the magnetic resonance data by at least performing a statistical analysis of a subset of a set of vasoreactivity measures. The subset is determined in accordance with the gas sensor data. The gas sensor data may for instance be used to determine when the concentration of carbon dioxide or for instance carbogen is elevated. This may be used to determine when the statistical analysis should be performed.

In another embodiment each vasoreactivity measure is calculated during the acquisition of magnetic resonance data. The instructions further cause the processor to perform the step of modifying the pulse sequence and/or the gas sequence in accordance with a set of vasoreactivity measures. The pulse sequence may be modified such that its contrast is changed to optimize the contrast versus the speed of the acquiring data period. For instance the repetition time and the number of echoes used may be changed. The gas sequence may also be modified in accordance with a set of vasoreactivity measures herein for instance if the vasoreactivity measure has reached a certain level of contrast in the vasoreactivity contrast images then the experiment can be terminated and the gas sequence can be ended and can be modified such that the subject begins breathing normal air again.

In another embodiment execution of the instructions further cause a processor to perform the step of modifying the pulse sequence and/or gas sequence in accordance with the gas sensor data. If the subject is not inhaling or exhaling an expected concentration of the gas then the gas sequence can be modified to correct this air. The pulse sequence can also be modified by using new gas sensor data. For instance, if the subject is not breathing an expected mixture of gas then the timing of the pulse sequence may be delayed a period.

In another embodiment the pulse sequence is a multi-gradient echo pulse sequence. This embodiment is advantageous because such a pulse sequence may be used to acquire images with different T2* weighted contrasts and to compute a quantitative T2* value from this set of images.

In another embodiment the magnetic resonance imaging system comprises the anesthesia system.

In another aspect the invention provides for a computer program product as defined in claim 9.

### BRIEF DESCRIPTION OF THE DRAWINGS

In the following preferred embodiments of the invention will be described, by way of example only, and with reference to the drawings in which:
Fig. 1 shows a block diagram which illustrates an an example useful for understanding the invention;
Fig. 2 shows a block diagram which illustrates a further example useful for understanding the invention;
Fig. 3 shows a block diagram which illustrates a further example useful for understanding the invention;
Fig. 4 shows a block diagram which illustrates a further example useful for understanding the invention;
Fig. 5 shows a magnetic resonance imaging system according to an embodiment of the invention;
Fig. 6 shows a magnetic resonance imaging system according to a further embodiment of the invention; and
Fig. 7 illustrates the effect of correct timing and incorrect for synchronization of the magnetic resonance data on a respiratory challenge experiment.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

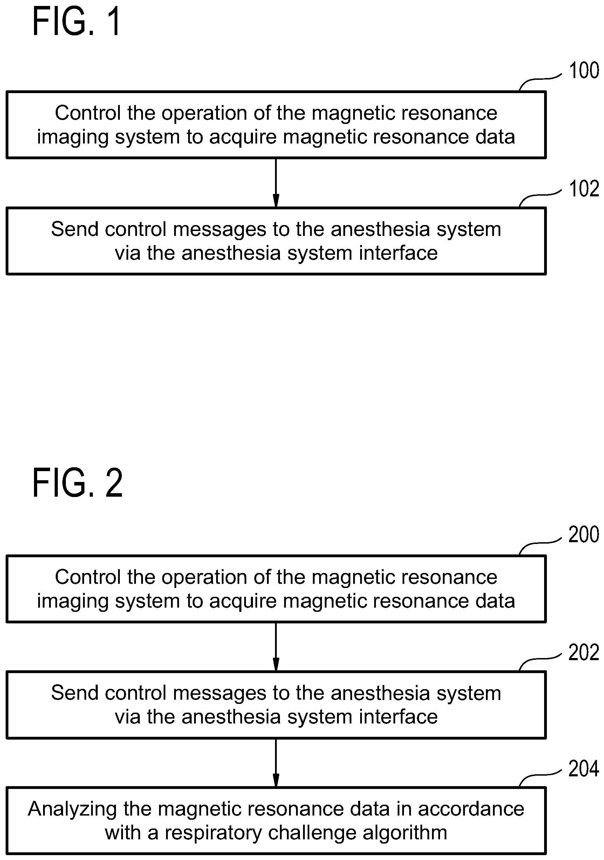

Fig. 1 shows a block diagram which illustrates a method useful for understanding the invention. In step 100 the operation of the magnetic resonance imaging system is controlled to acquire magnetic resonance data. In step 102 control messages are sent to the anesthesia system via the anesthesia system interface.

Fig. 2 shows a block diagram which illustrates a further method useful for understanding the invention. In step 200 the operation of the magnetic resonance imaging system is controlled in order to acquire magnetic resonance data. In step 202 control messages are sent to the anesthesia system via the anesthesia system interface. In step 204 the magnetic resonance data is analyzed in accordance with a respiratory challenge algorithm.

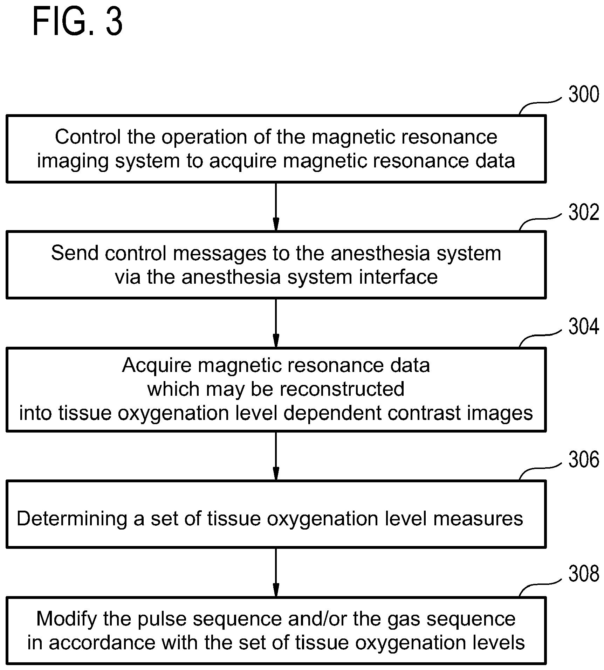

Fig. 3 shows a block diagram which illustrates a further method useful for understanding the invention. In step 300 the operation of the magnetic resonance imaging system is controlled in order to acquire magnetic resonance data. In step 302 control messages are sent to the anesthesia system via the anesthesia system interface. In step 304 magnetic resonance data is acquired which may be reconstructed into tissue oxygenation level dependent contrast images. The magnetic resonance data may be magnetic resonance data which contains T2* information. In step 306 a set of tissue oxygenation level measures are determined. Step 306 may include reconstructing the magnetic resonance data into tissue oxygenation level dependent contrast images And finally in step 308 the pulse sequence and/or the gas sequence is modified in accordance with a set of tissue oxygenation levels.

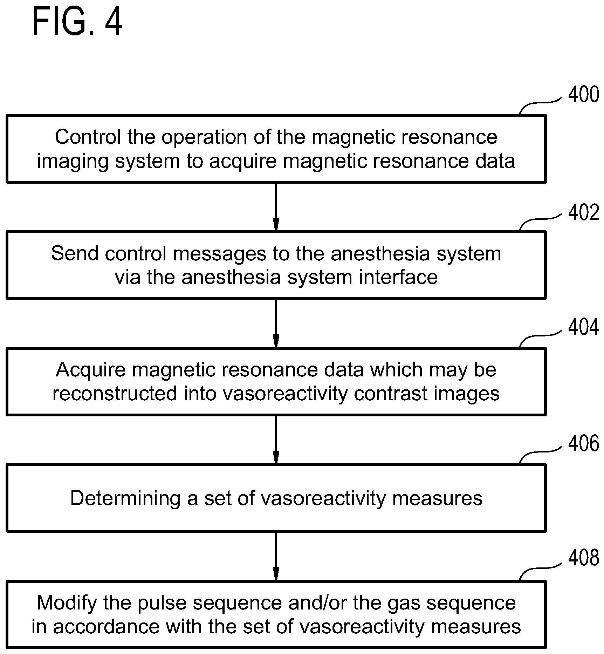

Fig. 4 shows a block diagram which illustrates a further method useful for understanding the invention. In step 400 the operation of the magnetic resonance imaging system is controlled in order to acquire magnetic resonance data. In step 402 control messages are sent to the anesthesia system via the anesthesia system interface. In step 404 magnetic resonance data is acquired which may be reconstructed into vasoreactivity contrast images. In step 406 a set of vasoreactivity measures is determined. Step 406 may include reconstructing the magnetic resonance data into vasoreactivity contrast images. In step 408 the pulse sequence and/or the gas sequence is modified in accordance with the set of vasoreactivity measures.

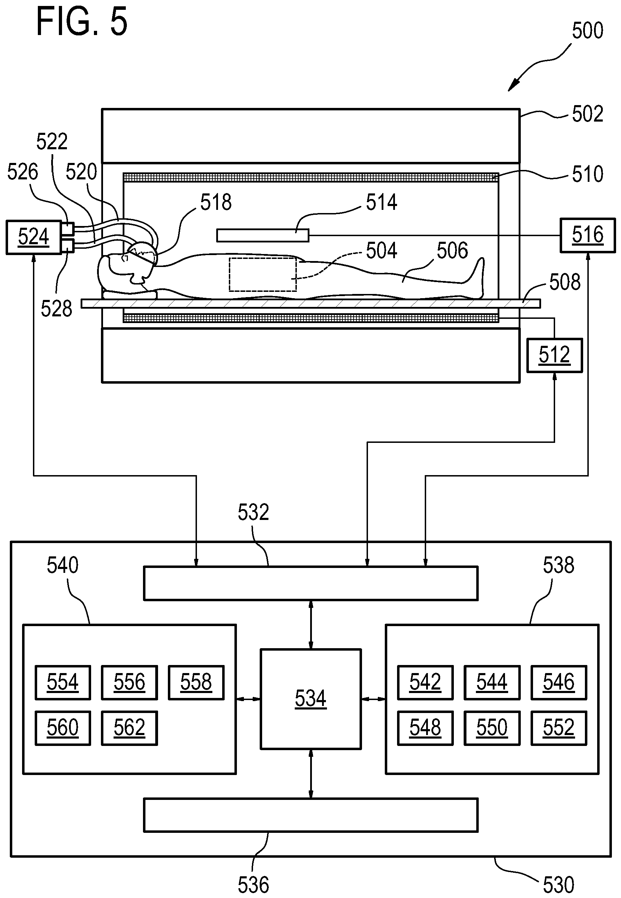

Fig. 5 shows a magnetic resonance imaging system 500 according to an embodiment of the invention. The magnetic resonance imaging system comprises a magnet 502 which is used to orient the position of magnetic spins within an imaging volume 504 of a subject 506 located within the bore of the magnet 502. The magnet 502 is shown as being a cylindrical type magnet with a bore through the center for receiving the subject. However other varieties of magnets could also be used such as so-called open or toroidal magnets. The subject 506 is shown as reposing on a subject support 508. Within the bore of the magnet 502 is a magnetic field gradient coil 510. The magnetic field gradient coil 510 is connected to a magnetic field gradient coil power supply 512. Magnetic resonance imaging systems typically have three separate coil systems. The gradient coil 510 and the gradient coil power supply 512 is intended to represent a standard gradient coil. Above the imaging volume 504 is a radio frequency coil 514. The radio frequency coil 514 is connected to a radio frequency transceiver 516. The combination of the radio frequency coil 514 and the radio frequency transceiver 516 is used to manipulate the orientation of magnetic spins within the imaging volume 504 and also to acquire magnetic resonance data by measuring the received radio signals.

The subject 506 is shown as having a mask 518 over his or her mouth. The mask 518 has an inlet tube 520 for receiving gas to the mask 518 and an outlet tube 522 for removing gas as it is exhaled by the subject 506. The inlet tube 520 and the outlet tube are connected to an anesthesia system 524. The anesthesia system 524 is able to control the mixture and amount of gases that are in the inlet tube 520 and are breathed by the subject 506. The inlet tube 520 is connected to the anesthesia system 524 through an inlet gas sensor 526. The outlet tube 522 is connected to the anesthesia system 524 through an outlet gas sensor 528. The inlet gas sensor and the outlet gas sensor 528 are for measuring a property of either the inlet gas in the inlet or outlet respectively. The inlet gas sensor 526 and the outlet gas sensor 528 generate gas sensor data. The anesthesia system 524 the gradient coil power supply 526 and the radio frequency transceiver 516 are all connected to a hardware interface 532 of a computer system 530. The hardware interface 532 may be considered to be an anesthesia system interface. The computer system controls all of these components.

The computer system 530 has a processor 534 which is connected to and controls the hardware interface 532 and a user interface 536. The user interface 536 may include devices for an operator to interact with and control the computer system 530. For instance the user interface 536 may also comprise a keyboard a common mouse and a display grid. Computer magnetic resonance images may be displayed on a display of the user interface 536. The processor 534 is also shown as being connected to computer memory 538 and computer storage 540.

The computer memory 508 is shown as containing a magnetic resonance imaging system control module 542. The magnetic resonance imaging system control module 542 contains executable code for controlling the operation of the magnetic resonance imaging system. The computer memory 538 is shown as further containing an anesthesia system control module. The anesthesia system control module 544 contains computer executable code for generating control messages for sending to the anesthesia system 524. The memory is further shown as containing a respiratory challenge protocol algorithm 546. The respiratory challenge protocol algorithm 546 contains computer executable code for analyzing magnetic resonance imaging data and/or images to analyze magnetic resonance imaging data acquired during a respiratory challenge protocol. The computer memory 538 is shown as further containing an image reconstruction module 548. The image reconstruction module 548 contains computer executable code for transforming magnetic resonance data 560 into magnetic resonance images. The computer memory 538 is further shown as containing a gas sequence modification module 550 and a pulse sequence modification module 552. The gas sequence modification module 550 contains computer executable code for modifying a gas sequence 556 using either magnetic resonance data 560 or gas sensor data 558. The pulse sequence modification module 552 contains computer executable code for modifying the pulse sequence 552 in accordance with either gas sensor data 558 or magnetic resonance data 560.

The computer storage 540 is shown as containing a pulse sequence 554 which is used in accordance with the magnetic resonance control system module 542 for controlling the magnetic resonance imaging system 500. The computer memory 540 is further shown as containing a gas sequence 556 which is used in accordance with the anesthesia system control module 544 for controlling the anesthesia system 524. The computer memory is further shown as containing gas sensor data 558 acquired using the inlet gas sensor 526 and/or the outlet gas sensor 528. The computer storage 540 is further shown as containing the magnetic resonance data 560 and a transverse plane relaxation time weighted magnetic resonance imaging image 562. The transverse plane relaxation time weighted magnetic resonance imaging image may be a T1, a T2, or a T2* weighted magnetic resonance imaging image.

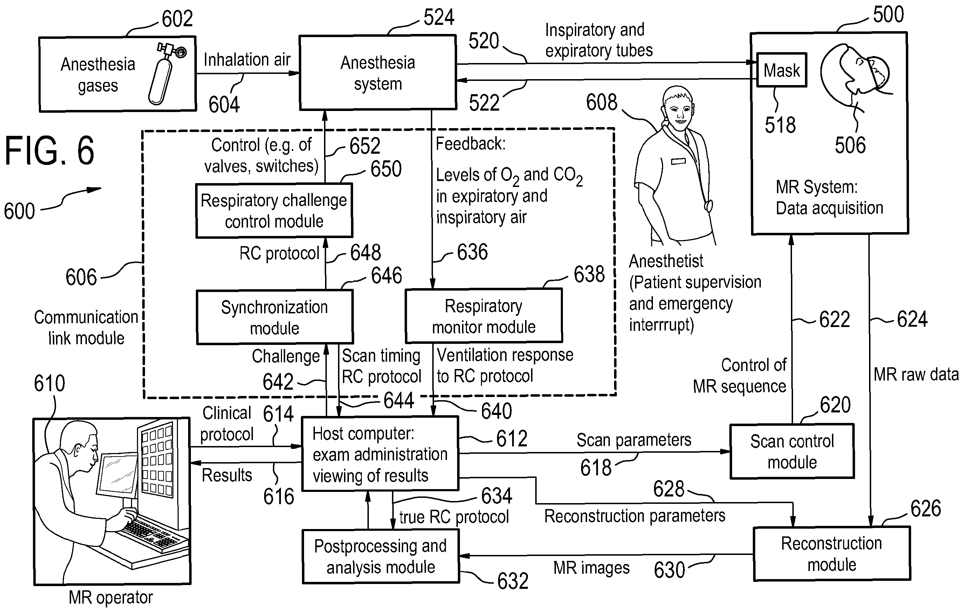

Fig. 6 is an example of a functional diagram 600 of the magnetic resonance imaging system 500 according to an embodiment of the invention. In figure 6 the control aspects of the system 600 are discussed. There is an anesthesia system which represents the control of anesthesia gases 602. The anesthesia system 504 receives inhalation air 604 from the anesthesia gases. Anesthesia system 524 controls the flow of a mixture of gases to a mask 518 via a inhalation tube 520 and receives them via an exhalation tube 522. Mask 518 is on a patient 506 within a magnetic resonance system 500. The anesthesia system 524 receives messages and sends messages to a host computer 612 via a communication link module 606. A magnetic resonance imaging system operator 610 is shown as controlling the clinical protocol 614 send to a host computer 612. The host computer 612 also generates results from the protocol 616 where they can be displayed for the operator 610. The host computer sends scan parameters 618 to a scan control module 620. The scan control module 620 is equivalent to the magnetic resonance imaging system control module 542 of figure 5. Scan control module 620 sends control signal 622 to the magnetic resonance imaging system 500. The raw magnetic resonance imaging data 624 is sent by the magnetic resonance imaging system 500 to an image reconstruction module 626. The reconstruction module 626 is equivalent to the image reconstruction module 548 of figure 5. The magnetic resonance raw data 624 is equivalent to the magnetic resonance data 560 at figure 5. The host computer 612 sends reconstruction parameters 628 to the reconstruction module 626.

The reconstruction module 626 sends the reconstructed magnetic resonance images 630 to a post processing and analysis module 632. The post processing and analysis module 632 receives true respiratory challenge protocol data 634 from the host computer 612. The true respiratory control protocol data was generated using data acquired from the communication link module 606.

The communication link module 606 performs a variety of functions. First the communication link module receives feedback levels 536 and oxygen and possibly carbon dioxide in expiratory and inspiratory air. The communication link module 606 is shown housing a respiratory monitor module 638 which receives the feedback levels 636. The respiratory monitor module sends ventilation response data for the respiratory control protocol 640 to the host computer 612. The host computer 642 sends data to a synchronization module 646 in the form of a challenge 642. The synchronization module 646 sends the respiratory control protocol 648 to a respiratory challenge control module 650. A synchronization module 646 also sends magnetic resonance imaging scan timing and respiratory challenge protocol data 644 to the host computer 612. Combination of the scan timing and respiratory challenge protocol 644 and the ventilation response to the respiratory control protocol 640 is used to generate the true respiratory challenge protocol data 634. The respiratory challenge control module 650 generates the control signals 652 which are sent to the anesthesia system 524 for controlling its operation.

Fig. 6 outlines a set-up for an MR monitored respiratory challenge. Conventionally, it contains more or less completely separated MR and RC environments, controlled independently by an MR (radiologist, MR technician) 610 and RC 608 operator (anesthetist), respectively.

The RC environment contains an anesthesia system 524 which comprises the arbitrary composition of inhalation gases 602 , delivery means 520, 522 for transporting the anesthesia mixture to the patient 506, delivery means to transport the inhaled and exhaled air to and from the patient via expiratory 520 and inspiratory 522 tubes, sensors that monitor gas levels in the inspiratory and expiratory tubes, a carbon dioxide absorber, means to prevent re-breathing of exhaled air, demand valves to optimize consumption of the delivered anesthesia, etc. The anesthesia system is supplied with inhalation gases such as Carbogen, CO2, pure oxygen or others from a gas supply unit, normally positioned outside the MR suite and the system is further able to introduce "normal" room air into the inhalation mixture. The inhalation mixture is delivered to the patient via an inspiratory tube 520 and a mask 518. The exhaled air is transported by a separate expiratory 522 tube from the patient to a scavenger system. Such an anesthesia system is normally controlled manually by an anesthetist, who further communicates abnormalities in the composition of inspiratory and/or expiratory air to the MR operator 610 to be considered in later post-processing.

The MR environment comprises a MR scanner 500 operated by a radiologist 610 or MR technician, who selects or even optimizes the scan protocol with respect to the clinical question. The selected scan protocol 614 is sent to the scan control module 620, which controls the MR machine 500. This timing of the scan protocol (e.g. the number and length of dynamics in a dynamic scan) requires synchronization with the RC protocol 648 (e.g. the MR and RC protocols need to start at the same time, the number of dynamic images needs to appropriately represent the MR signal changes during the experiment). The acquired data 624 are sent to the reconstruction module 626, reconstructing data, based on information and requirements determined by the scan protocol 614, selected by the MR operator.

The post-processing and analysis module 632 performs some preprocessing on the images 630 (e.g. motion correction, relaxometry, data filtering, etc.) and the quantitative analysis of the response (e.g. determination of the strength and kinetic of signal changes during oxygen breathing, etc.). The timing of the scan as well as the post-processing and analysis of the returned data may be manually synchronized with the breathing protocol 648 (e.g. the analysis module requires knowledge about the baseline and RC data to compare in statistical tests, signal models for numerical fits to the data depend on the timing of the RC protocol, etc).

The present invention involves a communication link module 606, which automates and synchronizes the communication between the MR scan and processing/analysis environment and the RC environment. This replaces the control function of the anesthetist 608. He is further necessary to supervise the patient and for possible security interrupts of the experiments.

In a preferred embodiment the communication link module consists of a synchronization module 646, a respiratory challenge control module 650, and a respiratory monitor module 638, described in more detail in the following paragraph. The communication link is driven by the scan administration environment on the MR host computer and controls an MR compatible anesthesia system 524.

In the embodiment shown in Fig. 6, the MR operator 610 selects a certain procedure with respect to a given clinical question. This contains the RC 648 to be applied (type of gas(es) and timing). The synchronization module adapts the RC to the MR scan and vice versa:
- It may adjusts the length of MR scans and the delivery and composition of particular inhalation mixtures: it ensures that the delivery does not change during the acquisition of an MR image, it synchronizes the length and the start time for both the MR and RC experiment, etc.,
- If required, it modifies scan parameters according to the particular challenge (e.g. it may select flow-insensitive imaging protocols if inhalation mixtures are used that have significant impact on blood flow, it may optimize the imaging protocol to optimize contrast with respect to the delivered inhalation mixture).
- The synchronization module 646 then sends the optimized MR protocol and the optimized RC protocol to the host computer and the respiratory challenge control module, respectively. The RC protocol is also provided to the post-processing/analysis module for later processing of the reconstructed data. The synchronization module can be realized as a piece of software, implemented on the scanner host.

The respiratory challenge control module 650 consists of a computer-readable storage medium that receives the RC protocol 648 from the synchronization module 646 and transforms it into programming instructions 652 to control the anesthesia system 524. Executed on a processor, it controls at least some of the valves of the anesthesia system to automatically select particular anesthesia gases and adjust the required gas pressures, flow, and compartments according to the RC protocol. It thus replaces the manual control of the anesthetist. In some embodiments, the respiratory challenge control unit is integrated in or attached to the anesthesia system.

The respiratory monitor module 638 reads out the monitoring sensors of the anesthesia system 524, which provide the patient respiratory signals (e.g. inspiratory and expiratory pressures of inhalation compartments). The patient respiratory signal is first represented by a graphical display and/or in a parameterized description. The representation may be provided by an LCD display integrated in the respiratory monitor module to be monitored by the anesthetist for security reasons. It may be useful to have a second representation sent to the MR host computer display for additional supervision by the MR operator (similar to how ECG signals are represented on the host computer display, conventionally). More importantly, the true patient respiratory signal, reflecting the true respiratory response to the challenge, translates into potential modifications of the RC protocol used in later post processing. The RC protocol is thus updated and sent to the post-processing/analysis module for later processing of the MR data. E.g., if the patient removes the mask after half of the experiment, the RC protocol and the signal model used by the analysis module would have to be shortened accordingly. The respiratory monitor module can also be realized as instructions contained on a computer-readable storage mediums, executed by the MR host computer 612 with communication to the sensors of the anaesthesia system, or it is integrated or attached to the anesthesia system, sending the monitoring results to the MR host computer, or it is part of the respiratory control unit with connections to both the MR and RC environment, etc.

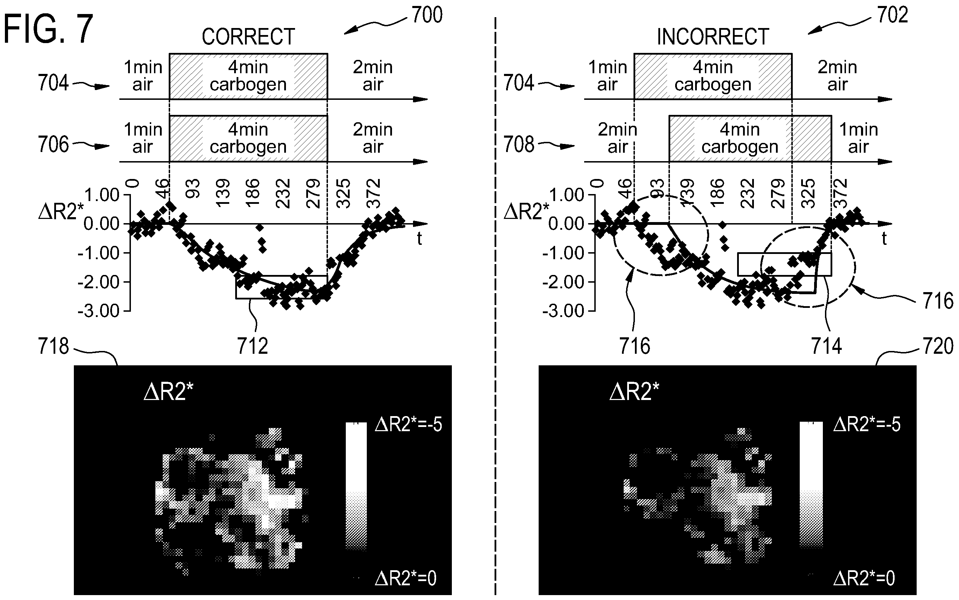

Fig. 7 is divided into two parts. The first part 700 illustrates correct timing 700 for synchronization of the magnetic resonance data and the respiratory challenge. The second part 702 illustrates incorrect timing between the analysis of the magnetic resonance imaging data and the timing of the respiratory challenge. In both halves 700, 702 there is a time line 704 which shows the intended timing for the respiratory challenge protocol. In this respiratory challenge protocol there is one minute air four minutes of carbogen gas and then two minutes of air is breathed by the subject. Time line 706 shows the actual respiratory challenge protocol performed with the correct timing 700 and time line 708 shows the actual respiratory challenge protocol performed when the timing was not synchronized. Time line 706 is identical with time line 704. However time line 708 is not identical with time line 704.

In time line 708 the subject breathes initially air for two minutes instead of one minute. The effect of this is that the carbogen gas is breathed by the subject one minute later than was expected. Below the time lines are graphs which show the change in R2* the time line is given in seconds in both cases. In the first half of figure 700 the box 712 indicates data points 712 that were used for performing the analysis. In the second half of the figure 702 the graph the box 714 indicates the data which was used for the statistical analysis. The graph in the second half 702 shows two regions 716 where the timing is off. In the first half 700 the data is used to reconstruct a delta R2* response map 718 for a map of a tumor showing meningioma. R2* is the reciprocal of T2*. A delta R2* response map is a graph which shows the change in R2*. For the above mentioned reading protocol. Using the shifted timing the same image 720 is calculated using the incorrect timing 702. A comparison of images 718 and 720 show that the contrast in image 718 is superior to that in 720.

Figure 7 shows the effect of miscommunication/wrong synchronization of the RC and MR data analysis protocols on the tumor response map and the model (right column). The left column depicts the outcome using the correct settings. The response map is based on a statistical test that compares periods of baseline breathing with the last 2min of the Carbogen challenge. Wrong synchronization of the respiratory challenge and the MR data analysis results in "darker" maps, since the response amplitude is underestimated as the test compares baseline data with the "wrong" data during Carbogen breathing. The model fit to the data fails due to wrong initialization of start and end points of the Carbogen challenge.

While the invention has been illustrated and described in detail in the drawings and foregoing description, such illustration and description are to be considered illustrative or exemplary and not restrictive; the invention is not limited to the disclosed embodiments and is defined solely by the appended claims.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 500: magnetic resonance imaging system
- 502: magnet
- 504: imaging volume
- 506: subject
- 508: subject support
- 510: magnetic field gradient coil
- 512: magnetic field gradient coil power supply
- 514: radio frequency coil
- 516: radio frequency transceiver
- 518: mask
- 520: inlet tube
- 522: outlet tube
- 524: anesthesia system
- 526: inlet gas sensor
- 528: outlet gas sensor
- 530: computer system
- 532: hardware interface
- 534: processor
- 536: user interface
- 538: computer memory
- 540: computer storage
- 542: magnetic resonance imaging system control module
- 544: anesthesia system control module
- 546: respiratory challenge protocol algorithm
- 548: image reconstruction module
- 550: gas sequence modification module
- 552: pulse sequence modification module
- 554: pulse sequence
- 556: gas sequence
- 558: gas sensor data
- 560: magnetic resonance data
- 562: transverse plan relaxation time weighted magnetic resonance imaging image
- 600: functional diagram
- 602: anesthesia gases
- 604: inhalation air
- 606: communications link module
- 608: anesthetist
- 610: operator
- 612: host computer
- 614: clinical protocol
- 616: results
- 618: scan parameters
- 620: scan control module
- 622: control of magnetic resonance sequence
- 624: magnetic resonance raw data
- 626: reconstruction module
- 628: reconstruction parameters
- 630: magnetic resonance image
- 632: post-processing and analysis module
- 634: true respiratory challenge protocol
- 636: feedback levels
- 638: respirator monitor module
- 640: ventilation response to respiratory challenge protocol
- 642: challenge
- 644: scan timing respiratory challenge protocol
- 646: synchronization module
- 648: respiratory challenge protocol
- 650: respiratory challenge control module
- 652: control commands
- 700: correct timing
- 702: incorrect timing
- 704: intended respiratory challenge protocol
- 706: actual respiratory challenge protocol
- 708: actual respiratory challenge protocol
- 712: box
- 714: box
- 716: region
- 718: delta R2* map
- 720: delta R2* map

## Claims

1. A magnetic resonance imaging system (500) comprising
- a magnet (502) adapted for generating a magnetic field for orientating the magnetic spins of nuclei of a subject located within an imaging volume (504);
- a radio frequency system (516) for acquiring magnetic resonance data,
wherein the radio frequency system comprises a radio frequency transceiver (516) adapted to connect to a radio frequency coil (514);
- a magnetic field gradient coil (510) for spatial encoding of the magnetic spins of nuclei within the imaging volume;
- a magnetic field gradient coil power supply (512) for supplying current to the magnetic field gradient coil;
- an anesthesia system interface (532) adapted for sending control messages to an anesthesia system (524), wherein the anesthesia system interface is further adapted for receiving gas sensor data (558, 636) from the anesthesia system, wherein the gas sensor data comprises time dependent gas concentrations in the gas inhaled and/or exhaled by the subject, wherein the gas sensor data is time correlated with the magnetic resonance data in accordance with a pulse sequence; and
- a computer system comprising a processor (534) and a memory (538, 540), wherein the memory contains instructions (542, 544, 546, 548, 550, 552) for execution by the processor, wherein execution of the instructions causes the processor to perform the steps of:
- controlling (100, 200, 300, 400) the operation of the magnetic resonance imaging system to acquire magnetic resonance data,
- sending (102, 202, 302, 402) control messages to the anesthesia system via the anesthesia system interface, and
wherein the computer memory contains said pulse sequence (554) for planning the acquisition of magnetic resonance data (560), wherein the instructions control the operation of the magnetic resonance imaging system in accordance with the pulse sequence,
wherein the memory comprises a gas sequence (556) for planning the temporal control of the composition of gas provided to the subject for respiration by the anesthesia system during the acquisition of magnetic resonance data according to a respiratory challenge, wherein execution of the instructions further cause the processor to perform the steps of:
acquiring (304) magnetic resonance data which may be reconstructed into tissue oxygenation level dependent contrast images, reconstructing the magnetic resonance data into tissue oxygenation level dependent contrast images (562), determining (306) a set of tissue oxygenation level measures, wherein the set of tissue oxygenation level measures is constructed by determining a tissue oxygenation measure for an oxygenation test volume in each of the tissue oxygenation level dependent contrast images, analyzing (204) the magnetic resonance data in accordance with a respiratory challenge algorithm (546) wherein the respiratory challenge algorithm analyzes the magnetic resonance data by at least performing a statistical analysis of a subset of the set of tissue oxygenation level measures, wherein the initialization of the respiratory challenge algorithm is performed in accordance with the gas sensor data and wherein the subset is determined in accordance with the gas sensor data, and
wherein the control messages (652) sent to the anesthesia system control the anesthesia gas delivery in accordance with the gas sequence, wherein the gas sequence is modified in accordance with the set of tissue oxygenation levels.

2. The magnetic resonance imaging system according to claim 1, wherein the gas concentrations measured in the gas comprise any one of the following: oxygen concentrations, carbon dioxide concentrations, and nitrogen concentrations.

3. The magnetic resonance imaging system according to any of claims 1 or 2, wherein each tissue oxygenation level measure is calculated during the acquisition of magnetic resonance data, wherein the instructions further cause the processor to perform the step of modifying the (308) pulse sequence in accordance with set of the tissue oxygen level measures.

4. The magnetic resonance imaging system according to any of claims 1-3, wherein execution of the instructions further cause the processor to perform the steps of:
- acquiring (404) magnetic resonance data which may be reconstructed into vasoreactivity contrast images (562);
- reconstructing the magnetic resonance data into vasoreactivity contrast images;
- determining (406) a set of vasoreactivity measures, wherein the set of vasoreactivity measures is constructed by determining a vasoreactivity level for a vasoreactivity test volume in each of the vasoreactivity contrast images;
wherein the respiratory challenge algorithm analyzes the magnetic resonance data by at least performing a statistical analysis of a subset of the set of vasoreactivity measures, and wherein the subset is determined in accordance with the gas sensor data.

5. The magnetic resonance imaging system according to claim 4, wherein each vasoreactivity measure is calculated during the acquisition of magnetic resonance data, wherein the instructions further cause the processor to perform the step of modifying (408) the pulse sequence and/or the gas sequence in accordance with the set of vasoreactivity measures.

6. The magnetic resonance imaging system according to any of claims 1-5, wherein execution of the instructions further cause the processor to perform the step of modifying the pulse sequence and/or gas sequence in accordance with the gas sensor data.

7. The magnetic resonance imaging system according to claim 3, wherein the pulse sequence is a multi-gradient echo pulse sequence.

8. The magnetic resonance imaging system according to any of the preceding claims, wherein the magnetic resonance imaging system comprises the anesthesia system.

9. A computer program product comprising machine readable instructions for execution by a processor of a computer system, wherein the computer system comprises a memory and is connected to a magnetic resonance imaging system, wherein the computer system is connected to an anesthesia system interface for sending control messages to an anesthesia system, wherein the anesthesia system interface is further adapted for receiving gas sensor data (558, 636) from the anesthesia system, wherein the gas sensor data comprises time dependent gas concentrations in the gas inhaled and/or exhaled by the subject, wherein the gas sensor data is time correlated with magnetic resonance data in accordance with a pulse sequence, wherein execution of the machine readable instructions causes the processor to perform the steps of:
- controlling (100, 200, 300, 400) the operation of the magnetic resonance imaging system; and
- sending (102, 202, 302, 402) control messages to the anesthesia system via the anesthesia system interface, and
wherein the computer memory contains said pulse sequence (554) for planning the acquisition of magnetic resonance data (560), wherein the instructions control the operation of the magnetic resonance imaging system in accordance with the pulse sequence,
wherein the memory comprises a gas sequence (556) for planning the temporal control of the composition of gas provided to the subject for respiration by the anesthesia system during the acquisition of magnetic resonance data according to a respiratory challenge,
wherein execution of the instructions further cause the processor to perform the steps of: acquiring (304) magnetic resonance data which may be reconstructed into tissue oxygenation level dependent contrast images, reconstructing the magnetic resonance data into tissue oxygenation level dependent contrast images (562), determining (306) a set of tissue oxygenation level measures, wherein the set of tissue oxygenation level measures is constructed by determining a tissue oxygenation measure for an oxygenation test volume in each of the tissue oxygenation level dependent contrast images, analyzing (204) the magnetic resonance data in accordance with a respiratory challenge algorithm (546) wherein the respiratory challenge algorithm analyzes the magnetic resonance data by at least performing a statistical analysis of a subset of the set of tissue oxygenation level measures, wherein the initialization of the respiratory challenge algorithm is performed in accordance with the gas sensor data and wherein the subset is determined in accordance with the gas sensor data, and wherein the control messages (652) sent to the anesthesia system control the anesthesia gas delivery in accordance with the gas sequence, wherein the gas sequence is modified in accordance with the set of tissue oxygenation levels.

## Patentansprüche

1. Magnetresonanz-Bildgebungssystem (500), umfassend:
einen Magneten (502), der zum Erzeugen eines Magnetfeldes zum Ausrichten der magnetischen Spins von Kernen eines Subjekts, das sich in einem Abbildungsvolumen (504) befindet, angepasst ist;
ein Hochfrequenzsystem (516) zum Erfassen von Magnetresonanzdaten, wobei das Hochfrequenzsystem einen Hochfrequenz-Transceiver (516) umfasst, der angepasst ist, um mit einer Hochfrequenzspule (514) zu verbinden;
eine Magnetfeldgradientenspule (510) zum räumlichen Codieren der der magnetischen Spins von Kernen innerhalb des Abbildungsvolumens;
eine Magnetfeldgradientenspulen-Stromversorgung (512) zum Liefern von Strom an die Magnetfeldgradientenspule;
eine Anästhesiesystemschnittstelle (532), die zum Senden von Steuernachrichten an ein Anästhesiesystem (524) angepasst ist, wobei die Anästhesiesystemschnittstelle weiter zum Empfangen von Gassensordaten (558, 636) von dem Anästhesiesystem angepasst ist, wobei die Gassensordaten zeitabhängige Gaskonzentrationen in dem von dem Subjekt eingeatmeten und/oder ausgeatmeten Gas umfassen, wobei die Gassensordaten mit den Magnetresonanzdaten gemäß einer Impulsfolge zeitlich korreliert sind; und
ein Computersystem, das einen Prozessor (534) und einen Speicher (538, 540) umfasst, wobei der Speicher Anweisungen (542, 544, 546, 548, 550, 552) zur Ausführung durch den Prozessor enthält, wobei die Ausführung der Anweisungen den Prozessor veranlasst, die Schritte auszuführen von:
- Steuern (100, 200, 300, 400) des Betriebs des Magnetresonanz-Bildgebungssystems, um Magnetresonanzdaten zu erfassen,
- Senden (102, 202, 302, 402) von Steuernachrichten an das Anästhesiesystem über die Anästhesiesystemschnittstelle, und
wobei der Computerspeicher die Impulsfolge (554) zum Planen der Erfassung von Magnetresonanzdaten (560) enthält, wobei die Anweisungen den Betrieb des Magnetresonanz-Bildgebungssystems gemäß der Impulsfolge steuern,
wobei der Speicher eine Gasfolge (556) zum Planen der zeitlichen Steuerung der Zusammensetzung des Gases umfasst, das dem Subjekt zur Atmung durch das Anästhesiesystem während der Erfassung von Magnetresonanzdaten gemäß einer respiratorischen Herausforderung bereitgestellt wird, und
wobei die Ausführung der Anweisungen weiter den Prozessor veranlasst, die Schritte auszuführen von:
Erfassen (304) von Magnetresonanzdaten, die in Gewebesauerstoffanreicherungsniveau abhängige Kontrastbilder rekonstruiert werden können, Rekonstruieren der Magnetresonanzdaten in Gewebesauerstoffanreicherungsniveau abhängige Kontrastbilder (562),
Bestimmen (306) eines Satzes von Gewebesauerstoffanreicherungs-niveaumaßnahmen, wobei der Satz von Gewebesauerstoffanreicherungs-niveaumaßnahmen durch ein Bestimmen einer Gewebesauerstoffanreicherungs-maßnahme für ein Sauerstoffanreicherungstestvolumen in jedem von den Gewebesauerstoffanreicherungsniveau abhängigen Kontrastbildern, Analysieren (204) der Magnetresonanzdaten gemäß eines Algorithmus (546) der respiratorischen Herausforderung, wobei der Algorithmus der respiratorischen Herausforderung die Magnetresonanzdaten durch zumindest ein Durchführen einer statistischen Analyse eines Teilsatzes des Satzes von Gewebesauer-stoffanreicherungsniveaumaßnahmen analysiert, wobei die Initialisierung des Algorithmus der respiratorischen Herausforderung gemäß den Gassensordaten durchgeführt wird und wobei der Teilsatz gemäß den Gassensordaten bestimmt wird, und
wobei die an das Anästhesiesystem gesendeten Steuernachrichten (652) die Anästhesiegaszufuhr gemäß der Gasfolge steuern, wobei die Gasfolge gemäß dem Satz von Gewebesauerstoffanreicherungsniveaus modifiziert wird.

2. Magnetresonanz-Bildgebungssystem nach Anspruch 1, wobei die in dem Gas gemessenen Gaskonzentrationen eines der Folgenden umfassen: Sauerstoff-konzentrationen, Kohlendioxidkonzentrationen und Stickstoffkonzentrationen.

3. Magnetresonanz-Bildgebungssystem nach einem der Ansprüche 1 oder 2, wobei jede Gewebesauerstoffanreicherungsmaßnahme während dem Erfassen der Magnetresonanzdaten berechnet wird, wobei die Anweisungen weiter den Prozessor veranlassen, den Schritte des Modifizierens (308) der Impulsfolge gemäß dem Satz der Gewebesauerstoffanreicherungsniveaumaßnahmen durchzuführen.

4. Magnetresonanz-Bildgebungssystem nach einem der Ansprüche 1-3, wobei die Ausführung der Anweisungen den Prozessor veranlasst, die Schritte auszuführen von:
- Erfassen (404) von Magnetresonanzdaten, die in Vasoreaktivitäts-kontrastbilder (562) rekonstruiert werden können;
- Rekonstruieren der Magnetresonanzdaten in Vasoreaktivitäts-kontrastbilder;
- Bestimmen (406) eines Satzes von Vasoreaktivitätsmaßnahmen, wobei der Satz von Vasoreaktivitätsmaßnahmen durch Bestimmen eines Vasoreaktivitäts-niveaus für ein Vasoreaktivitätstestvolumen in jedem der Vasoreaktivitäts-kontrastbilder konstruiert wird;
wobei der wobei der Algorithmus der respiratorischen Herausforderung die Magnetresonanzdaten durch zumindest ein Durchführen einer statistischen Analyse eines Teilsatzes des Satzes von Vasoreaktivitätsmaßnahmen analysiert, und wobei der Teilsatz gemäß den Gassensordaten bestimmt wird.

5. Magnetresonanz-Bildgebungssystem nach Anspruch 4, wobei jede Vasoreaktivitätsmaßnahme während der Erfassung von Magnetresonanzdaten berechnet wird, wobei die Anweisungen weiter den Prozessor veranlassen, den Schritte des Modifizierens (408) der Impulsfolge und/oder der Gasfolge gemäß dem Satz der Vasoreaktivitätsmaßnahmen durchzuführen.

6. Magnetresonanz-Bildgebungssystem nach einem der Ansprüche 1-5, wobei die Ausführung der Anweisungen weiter den Prozessor veranlasst, den Schritt des Modifizierens der impulsfolge und/oder der Gasfolge gemäß den Gassensordaten durchzuführen.

7. Magnetresonanz-Bildgebungssystem nach Anspruch 3, wobei die Impulsfolge eine Mehrgradienten-Echo-Impulsfolge ist.

8. Magnetresonanz-Bildgebungssystem nach einem der vorstehenden Ansprüche, wobei das Magnetresonanz-Bildgebungssystem das Anästhesiesystem umfasst.

9. Computerprogrammprodukt, umfassend maschinenlesbare Anweisungen zur Ausführung durch einen Prozessor eines Computersystems, wobei das Computersystem einen Speicher umfasst und mit einem Magnetresonanz-Bildgebungssystem verbunden ist, wobei das Computersystem zum Senden von Steuernachrichten an ein Anästhesiesystem mit einer Anästhesiesystem-schnittstelle verbunden ist, wobei die Anästhesiesystemschnittstelle weiter zum Empfangen von Gassensordaten (558, 636) von dem Anästhesiesystem angepasst ist, wobei die Gassensordaten zeitabhängige Gaskonzentrationen in dem von dem Subjekt eingeatmeten und/oder ausgeatmeten Gas umfassen, wobei die Gassensordaten mit den Magnetresonanzdaten gemäß einer Impulsfolge zeitlich korreliert sind, wobei eine Ausführung der maschinenlesbaren Anweisungen den Prozessor veranlasst, die Schritte auszuführen von:
- Steuern (100, 200, 300, 400) des Betriebs des Magnetresonanz-Bildgebungssystems; und
- Senden (102, 202, 302, 402) von Steuernachrichten an das Anästhesiesystem über die Anästhesiesystemschnittstelle, und
wobei der Computerspeicher die Impulsfolge (554) zum Planen der Erfassung von Magnetresonanzdaten (560) enthält, wobei die Anweisungen den Betrieb des Magnetresonanz-Bildgebungssystems gemäß der Impulsfolge steuern,
wobei der Speicher eine Gasfolge (556) zum Planen der zeitlichen Steuerung der Zusammensetzung des Gases umfasst, das dem Subjekt zur Atmung durch das Anästhesiesystem während der Erfassung von Magnetresonanzdaten gemäß einer respiratorischen Herausforderung bereitgestellt wird,
wobei die Ausführung der Anweisungen weiter den Prozessor veranlasst, die Schritte auszuführen von:
Erfassen (304) von Magnetresonanzdaten, die in Gewebesauerstoff-anreicherungsniveau abhängige Kontrastbilder rekonstruiert werden können, Rekonstruieren der Magnetresonanzdaten in Gewebesauerstoffanreicherungs-niveau abhängige Kontrastbilder (562), Bestimmen (306) eines Satzes von Gewebesauerstoffanreicherungsniveaumaßnahmen, wobei der Satz von Gewebesauerstoffanreicherungsniveaumaßnahmen durch ein Bestimmen einer Gewebesauerstoffanreicherungsmaßnahme für ein Sauerstoffanreicherungstestvolumen in jedem von den Gewebesauerstoffanreicherungsniveau abhängigen Kontrastbildern, Analysieren (204) der Magnetresonanzdaten gemäß eines Algorithmus (546) der respiratorischen Herausforderung, wobei der Algorithmus der respiratorischen Herausforderung die Magnetresonanzdaten durch zumindest ein Durchführen einer statistischen Analyse eines Teilsatzes des Satzes von Gewebesauerstoffanreicherungsniveaumaßnahmen analysiert, wobei die Initialisierung des Algorithmus der respiratorischen Herausforderung gemäß den Gassensordaten durchgeführt wird und wobei der Teilsatz gemäß den Gassensordaten bestimmt wird, und
wobei die an das Anästhesiesystem gesendeten Steuernachrichten (652) die Anästhesiegaszufuhr gemäß der Gasfolge steuern,
wobei die Gasfolge gemäß dem Satz von Gewebesauerstoffanreicherungs-niveaus modifiziert wird.

## Revendications

1. Système d'imagerie à résonance magnétique (500) comprenant :
- un aimant (502) qui est à même de générer un champ magnétique pour orienter les spins magnétiques de noyaux d'un sujet situé au sein d'un volume d'imagerie (504) ;
- un système de fréquence radio (516) pour acquérir des données de résonance magnétique, dans lequel le système de fréquence radio comprend un émetteur-récepteur de fréquence radio (516) qui est à même de se connecter à une bobine de fréquence radio (514) ;
- une bobine de gradient de champ magnétique (510) pour un codage spatial des spins magnétiques de noyaux au sein du volume d'imagerie ;
- une alimentation électrique de bobine de gradient de champ magnétique (512) pour fournir du courant à la bobine de gradient de champ magnétique ;
- une interface de système d'anesthésie (532) qui est à même d'envoyer des messages de commande à un système d'anesthésie (524), dans lequel l'interface de système d'anesthésie est en outre à même de recevoir des données de capteur de gaz (558, 636) du système d'anesthésie, dans lequel les données de capteur de gaz comprennent des concentrations de gaz en fonction du temps dans le gaz inhalé et/ou exhalé par le sujet, dans lequel les données de capteur de gaz sont corrélées dans le temps avec les données de résonance magnétique conformément à une séquence d'impulsions ; et
- un système d'ordinateur comprenant un processeur (534) et une mémoire (538, 540), dans lequel la mémoire contient des instructions (542, 544, 546, 548, 550, 552) pour exécution par le processeur, dans lequel l'exécution des instructions amène le processeur à effectuer les étapes consistant à :
- commander (100, 200, 300, 400) le fonctionnement du système d'imagerie à résonance magnétique pour acquérir des données de résonance magnétique,
- envoyer (102, 202, 302, 402) des messages de commande au système d'anesthésie via l'interface du système d'anesthésie et
dans lequel la mémoire d'ordinateur contient ladite séquence d'impulsions (554) pour planifier l'acquisition de données de résonance magnétique (560), dans lequel les instructions commandent le fonctionnement du système d'imagerie à résonance magnétique conformément à la séquence d'impulsions,
dans lequel la mémoire comprend une séquence de gaz (556) pour planifier la commande temporelle de la composition de gaz fournie au sujet pour la respiration par le système d'anesthésie au cours de l'acquisition de données de résonance magnétique conformément à une épreuve respiratoire,
dans lequel l'exécution des instructions amène en outre le processeur à effectuer les étapes consistant à :
acquérir (304) des données de résonance magnétique qui peuvent être reconstruites en images de contraste dépendant du niveau d'oxygénation des tissus, reconstruire les données de résonance magnétique en images de contraste (562) dépendant du niveau d'oxygénation des tissus, déterminer (306) un ensemble de mesures de niveau d'oxygénation des tissus, dans lequel l'ensemble de mesures de niveau d'oxygénation des tissus est conçu en déterminant une mesure d'oxygénation des tissus pour un volume d'essai d'oxygénation dans chacune des images de contraste dépendant des niveaux d'oxygénation des tissus, analyser (204) les données de résonance magnétique avec un algorithme d'épreuve respiratoire (546), dans lequel l'algorithme d'épreuve respiratoire analyse les données de résonance magnétique en effectuant au moins une analyse statistique d'un sous-ensemble de l'ensemble de mesures de niveaux d'oxygénation des tissus, dans lequel l'initialisation de l'algorithme d'épreuve respiratoire est effectuée conformément aux données du capteur de gaz
et dans lequel le sous-ensemble est déterminé conformément aux données du capteur de gaz, et dans lequel les messages de commande (652) envoyés au système d'anesthésie commandent la fourniture de gaz anesthésiant conformément à la séquence de gaz, dans lequel la séquence de gaz est modifiée conformément à l'ensemble de niveaux d'oxygénation des tissus.

2. Système d'imagerie à résonance magnétique selon la revendication 1, dans lequel les concentrations de gaz mesurées dans le gaz comprennent l'une quelconque des suivantes : concentrations d'oxygène, concentrations d'anhydride carbonique et concentrations d'azote.

3. Système d'imagerie à résonance magnétique selon l'une quelconque des revendications 1 ou 2, dans lequel chaque mesure de niveau d'oxygénation des tissus est calculé au cours de l'acquisition de données de résonance magnétique, dans lequel les instructions amènent en outre le processeur à effectuer l'étape de modification (308) de la séquence d'impulsions conformément à l'ensemble de mesures de niveau d'oxygène des tissus.

4. Système d'imagerie à résonance magnétique selon l'une quelconque des revendications 1-3, dans lequel l'exécution des instructions amène en outre le processeur à effectuer les étapes consistant à :
- acquérir (404) des données de résonance magnétique qui peuvent être reconstruites en images de contraste de vasoréactivité (562) ;
- reconstruire les données de résonance magnétique en images de contraste de vasoréactivité ;
- déterminer (406) un ensemble de mesures de vasoréactivité, dans lequel l'ensemble de mesures de vasoréactivité est construit en déterminant un niveau de vasoréactivité pour un volume d'essai de vasoréactivité dans chacune des images de contraste de vasoréactivité ;
dans lequel l'algorithme d'épreuve respiratoire analyse les données de résonance magnétique en effectuant au moins une analyse statistique d'un sous-ensemble de l'ensemble de mesures de vasoréactivité, et dans lequel le sous-ensemble est déterminé conformément aux données du capteur de gaz.

5. Système d'imagerie à résonance magnétique selon la revendication 4, dans lequel chaque mesure de vasoréactivité est calculée au cours de l'acquisition de données de résonance magnétique, dans lequel les instructions amènent en outre le processeur à effectuer l'étape de modification (408) de la séquence d'impulsions et/ou de la séquence de gaz conformément à l'ensemble de mesures de vasoréactivité.

6. Système d'imagerie à résonance magnétique selon l'une quelconque des revendications 1-5, dans lequel l'exécution des instructions amène en outre le processeur à effectuer l'étape de modification de la séquence d'impulsions et/ou de la séquence de gaz conformément aux données du capteur de gaz.

7. Système d'imagerie à résonance magnétique selon la revendication 3, dans lequel la séquence d'impulsions est une séquence d'impulsions à écho multigradient.

8. Système d'imagerie à résonance magnétique selon l'une quelconque des revendications précédentes, dans lequel le système d'imagerie à résonance magnétique comprend le système d'anesthésie.

9. Produit de programme d'ordinateur comprenant des instructions lisibles par ordinateur pour exécution par un processeur d'un système d'ordinateur, dans lequel le système d'ordinateur comprend une mémoire et est connecté à un système d'imagerie à résonance magnétique, dans lequel le système d'ordinateur est connecté à une interface de système d'anesthésie pour envoyer des messages de commande à un système d'anesthésie, dans lequel l'interface de système d'anesthésie est en outre à même de recevoir des données de capteur de gaz (558, 636) depuis le système d'anesthésie, dans lequel les données du capteur de gaz sont corrélées dans le temps avec les données de résonance magnétique conformément à une séquence d'impulsions, dans lequel l'exécution des instructions lisibles par ordinateur amène le processeur à effectuer les étapes consistant à :
- commander (100, 200, 300, 400) le fonctionnement du système d'imagerie à résonance magnétique ;
- envoyer (102, 202, 302, 402) des messages de commande au système d'anesthésie via l'interface du système d'anesthésie et
dans lequel la mémoire d'ordinateur contient ladite séquence d'impulsions (554) pour planifier l'acquisition de données de résonance magnétique (560), dans lequel les instructions commandent le fonctionnement du système d'imagerie à résonance magnétique conformément à la séquence d'impulsions,
dans lequel la mémoire comprend une séquence de gaz (556) pour planifier la commande temporelle de la composition de gaz fournie au sujet pour la respiration par le système d'anesthésie au cours de l'acquisition de données de résonance magnétique conformément à une épreuve respiratoire,
dans lequel l'exécution des instructions amène en outre le processeur à effectuer les étapes consistant à acquérir (304) des données de résonance magnétique qui peuvent être reconstruites en images de contraste dépendant du niveau d'oxygénation des tissus, reconstruire les données de résonance magnétique en images de contraste (562) dépendant du niveau d'oxygénation des tissus, déterminer (306) un ensemble de mesures de niveau d'oxygénation des tissus, dans lequel l'ensemble de mesures de niveau d'oxygénation des tissus est conçu en déterminant une mesure d'oxygénation des tissus pour un volume d'essai d'oxygénation dans chacune des images de contraste dépendant des niveaux d'oxygénation des tissus, analyser (204) les données de résonance magnétique avec un algorithme d'épreuve respiratoire (546), dans lequel l'algorithme d'épreuve respiratoire analyse les données de résonance magnétique en effectuant au moins une analyse statistique d'un sous-ensemble de l'ensemble de mesures de niveaux d'oxygénation des tissus, dans lequel l'initialisation de l'algorithme d'épreuve respiratoire est effectuée conformément aux données du capteur de gaz et dans lequel le sous-ensemble est déterminé conformément aux données du capteur de gaz, et dans lequel les messages de commande (652) envoyés au système d'anesthésie commandent la fourniture de gaz anesthésiant conformément à la séquence de gaz, dans lequel la séquence de gaz est modifiée conformément à l'ensemble de niveaux d'oxygénation des tissus.
